(19) Europäisches Patentamt
European Patent Office
Office européen des brevets

(11) EP 3 124 006 A1

(12) EUROPEAN PATENT APPLICATION
published in accordance with Art. 153(4) EPC

(43) Date of publication:
01.02.2017 Bulletin 2017/05

(51) Int Cl.:
A61J 1/10 (2006.01)

(21) Application number: 15769544.6

(22) Date of filing: 18.02.2015

(86) International application number:
PCT/JP2015/054445

(87) International publication number:
WO 2015/146382 (01.10.2015 Gazette 2015/39)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 26.03.2014 JP 2014064121

(71) Applicant: Terumo Kabushiki Kaisha
Tokyo 151-0072 (JP)

(72) Inventors:
• ICHIKAWA, Saki
Fujinomiya-shi
Shizuoka 418-0004 (JP)
• TAKEDA, Norihiko
Fujinomiya-shi
Shizuoka 418-0004 (JP)
• KORA, Shinichi
Fujinomiya-shi
Shizuoka 418-0004 (JP)

(74) Representative: Casalonga
Casalonga & Partners
Bayerstraße 71/73
80335 München (DE)

(54) **BLOOD RESERVOIR**

(57) A blood storage container includes a container main body made of a resin composition incorporating, per 100 parts by mass of polyvinyl chloride, 30 to 70 parts by mass of a composite plasticizer combining a first plasticizer and a second plasticizer, the first plasticizer being di-isononyl-cyclohexane-1,2-dicarboxylate, the second plasticizer being a citrate, the blending ratio between the first plasticizer and the second plasticizer in the composite plasticizer being 15/85 to 93/7.

**Description**

Technical Field

The present invention relates to a blood storage container used for the storage of blood, particularly a red blood cell concentrate.

Background Art

Typical examples of materials to form a blood storage container include polyvinyl chloride (PVC) containing a plasticizer such as diethylhexyl phthalate (DEHP). Even though the generation of dioxin at the time of disposal, etc., has been regarded as a problem, PVC is still the mainstream of materials for a blood storage container, especially red blood cell storage container, even now. The biggest reason therefor is that during the storage of a red blood cell concentrate, DEHP contained in PVC leaches into the red blood cell concentrate, thereby suppressing the morphological change or (hemolysis). However, according to the Registration, Evaluation, Authorization and Restriction of Chemicals (REACH) that has recently started in Europe, DEHP is listed as one of substances of very high concern because, depending on the amount, it may cause endocrine disruption (reproductive toxicity), etc. For this reason, as a material to form a blood storage container, the development of a PVC composition not containing DEHP as a plasticizer has been desired.
Various proposals have been made as PVC compositions to form a blood storage container. For example, Patent Literature 1 describes a PVC composition incorporating a citrate, and it is stated that a storage bag made of the PVC composition has excellent storage stability for whole blood and platelets.

Citation List

Patent Literature

Patent Literature 1: JP 61-51044 A

Summary of Invention

Technical Problem

However, in the actual situation, in the storage of blood, particularly red blood cells, the PVC composition described in Patent Literature 1 has not reached a fully satisfactory level in terms of the suppressing effect on (hemolysis), and its storage stability for red blood cells is poor.
The present invention has been accomplished against the above background, and is addressed to the problem of providing a blood storage container made of a PVC composition not containing DEHP as a plasticizer, which has excellent storage stability for blood, particularly red blood cells.

Solution to Problem

To solve the above problem, a blood storage container according to the present invention includes a container main body made of a resin composition incorporating, per 100 parts by mass of polyvinyl chloride, 30 to 70 parts by mass of a composite plasticizer combining a first plasticizer and a second plasticizer, the first plasticizer being di-isononyl-cyclohexane-1,2-dicarboxylate (DiNCH), the second plasticizer being a citrate, the blending ratio between the first plasticizer and the second plasticizer in the composite plasticizer being 15/85 to 93/7. Further, in the blood storage container of the present invention, the citrate is preferably butyryl trihexyl citrate (BTHC).
In the above configuration, the container main body is made of a resin composition incorporating polyvinyl chloride and a predetermined amount of composite plasticizer combining DiNCH and a citrate in a predetermined blending ratio. As a result, the storage stability for blood, particularly blood cells, placed inside the container main body is improved. Particularly when a red blood cell concentrate is stored, hemolysis is suppressed. In addition, because the container main body is made of a resin composition incorporating a predetermined amount of composite plasticizer, the pressure resistance of the container main body is also maintained.

Advantageous Effects of Invention

The present invention makes it possible to provide a blood storage container that is made of a PVC composition not containing DEHP as a plasticizer, has excellent storage stability for blood, particularly blood cells (among blood cells, red blood cells), and is also excellent in terms of the pressure resistance of the container.

Brief Description of Drawings

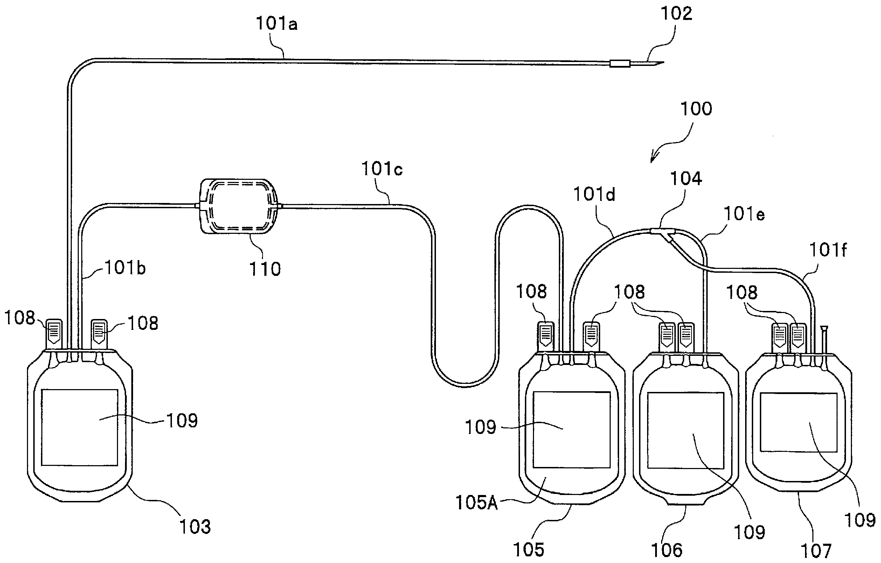

Fig. 1 is a schematic diagram showing the configuration of a blood bag system using the blood storage container according to the present invention.

Description of Embodiments

Embodiments of the blood storage container according to the present invention will be described.

The blood storage container of the present invention includes a container main body made of a resin composition incorporating, relative to polyvinyl chloride, a predetermined amount of composite plasticizer combining a first plasticizer and a second plasticizer in a predetermined blending ratio. The first plasticizer is di-isononyl-cyclohexane-1,2-dicarboxylate (DiNCH), and the second plasticizer is a citrate. In addition, the citrate is butyryl trihexyl citrate (BTHC), acetyl trihexyl citrate (ATHC), or the like, and BTHC is preferable.

The resin composition incorporates 30 to 70 parts by mass of the composite plasticizer per 100 parts by mass of polyvinyl chloride. When the amount of composite plasticizer incorporated relative to polyvinyl chloride is less than 30 parts by mass, the resulting resin composition is hard, making it difficult to shape the container main body. In addition, when the amount of composite plasticizer incorporated is more than 70 parts by mass, although the storage stability for blood, specifically red blood cells, placed inside the container main body is excellent, the pressure resistance of the container main body decreases. Incidentally, it is more preferable that the amount of composite plasticizer incorporated per 100 parts by mass of polyvinyl chloride is 40 to 60 parts by mass.

The blending ratio between the first plasticizer and the second plasticizer in the composite plasticizer is 15/85 to 93/7. That is, the amount of DiNCH incorporated, which is the first plasticizer in the composite plasticizer, is 15 to 93 mass%, while the amount of citrate incorporated (the remainder), which is the second plasticizer, is 7 to 85 mass%. Incidentally, it is more preferable that the blending ratio is within a range of 29/71 to 71/29.

When the amount of DiNCH incorporated is less than 15 mass%, that is, when the amount of citrate incorporated, the remainder, is more than 85 mass%, although the pressure resistance of the container main body is maintained, the hemolysis rate of blood, specifically red blood cells, placed inside the container main body increases, resulting in a decrease in the storage stability for red blood cells.

In addition, when the amount of DiNCH incorporated is more than 93 mass%, that is, when the amount of citrate incorporated, the remainder, is less than 7 mass%, although the pressure resistance of the container main body is maintained, the hemolysis rate of blood, specifically red blood cells, placed in the container main body increases, resulting in a decrease in the storage stability for red blood cells.

In addition to the polyvinyl chloride, DiNCH, and citrate described above, it is preferable that the resin composition also incorporates heat stabilizers such as epoxidized soybean oil, zinc soap, and calcium soap. With respect to the amounts of heat stabilizers incorporated, it is preferable that, for example, per 100 parts by mass of polyvinyl chloride, epoxidized soybean oil is 4 to 16 parts by mass, zinc soap is 0.01 to 1 part by mass, and calcium soap is 0.01 to 1 part by mass.

The shape of the blood storage container is not particularly limited, but is preferably a bag shape as shown in Fig. 1. In addition, it is preferable that the volume of the blood storage container 105, that is, the volume of the container main body 105A, is 100 to 600 mL. In addition, the container main body 105A may also be provided with, for example, discharge ports 108 that communicate with the inside of the blood storage container 105 and are used for the discharge of blood components (red blood cell concentrate, etc.) placed inside the container main body 105A, for example, or tubes 101c and 101d for connection with other containers (bags) or the like. Then, as the discharge port 108 and the tubes 101c and 101d, those used in conventional blood storage containers are used, and they are made of polyvinyl chloride or the like.

The blood storage container 105 is produced by a conventionally known method as follows.

First, the above resin composition is extruded through a T-die or a circular die, and, suitably utilizing a technique such as thermoforming, blowing, stretching, cutting, or fusing, the obtained flat sheet, tubular sheet, parison, or the like is formed into a bag-shaped container main body 105A to give a blood storage container 105. It is preferable that two flat sheets are fused together to produce a bag-shaped container main body 105A to give a blood storage container 105. The thickness of the container main body 105A, that is, the thickness of the sheet, is preferably 0.05 to 1.0 mm, and still more preferably 0.08 to 0.8 mm. In addition, as necessary, during or after the production of the container main body 105A, the discharge port 108 and the tubes 101c and 101d are joined to the container main body 105A.

During the storage of red blood cells (red blood cell concentrate) in the blood storage container 105, it is preferable that the inside of the container main body 105A is filled with a red blood cell storage solution. In addition, as necessary, a hemolysis inhibitor, a surfactant, or the like may also be added to the red blood cell storage solution.

Incidentally, hereinafter, a red blood cell concentrate having added thereto a red blood cell storage solution, a hemolysis inhibitor, an additive, or the like is referred to as "red blood cell preparation".

As the red blood cell storage solution, known storage solutions used for the long-term storage of red blood cell concentrates are usable. Examples of red blood cell storage solutions include ACD solution, CPD solution, MAP solution, SAGM solution, OPTISOL (registered trademark) (AS-5), ADSOL (AS-1), Nutricel (AS-3), PAGG-S, and SAGP-maltose. Incidentally, it is preferable that the amount of red blood cell storage solution added is 40 to 60 mL per 100 mL of the red blood cell concentrate, that is, 20 to 30 mL per 100 mL of sampled blood.

A hemolysis inhibitor serves to prevent the destruction of the red blood cell membrane owing to its antioxidant effect, the affinity between the red blood cell membrane and lipid, and the like. Specifically, vitamin E (including acetate compounds, such as tocopherol) and unsaturated chain hydrocarbon compounds, such as 7-tetradecene, 8-octadecene, 9-eicosene, and squalene, are preferable. It is preferable that the amount of hemolysis inhibitor added is 25 to 100 ppm as a concentration in the red blood cell preparation.

A surfactant has the function of promoting the uniform dispersion of a hemolysis inhibitor into a red blood cell storage solution and also the covering of the red blood cell membrane with a hemolysis inhibitor, and can also suppress the destruction of the red blood cell membrane by itself. Specifically, polyoxyethylene oleyl ether (EMULGEN (registered trademark) 430), polyoxyethylene sorbitan monooleate (TWEEN (registered trademark) 80), and the like are preferable. It is preferable that the amount of surfactant added is 100 to 300 ppm as a concentration in the red blood cell preparation.

Next, a blood bag system using the blood storage container according to the present invention will be described. As shown in Fig. 1, the blood storage container is equivalent to the blood storage bag 105 of the blood bag system 100.

The blood bag system 100 includes: a blood sampling bag 103 to be filled with blood sampled from the blood donor through a blood sampling tube 101a having a blood sampling needle 102 at its end; a blood processing filter 110 for separating predetermined blood components (white blood cells and platelets) from the blood (whole blood) transferred through a tube 101b from the blood sampling bag 103; a blood storage bag 105 for recovering the blood which has passed through the blood processing filter 110 through the tube 101c and from which predetermined blood components have thus been removed; a blood storage bag 106 to which the upper-layer blood component (blood plasma) prepared by centrifugation in the blood storage bag 105 is transferred through the tubes 101d and 101e and a branch pipe 104; and a medical-solution-filled bag 107 filled with a red blood cell storage solution or the like. The red blood cell storage solution or the like is transferred from the medical-solution-filled bag 107 through the tubes 101f and 101d and the branch pipe 104 to the blood storage bag 105, and added to the lower-layer blood component (red blood cell concentrate) prepared by centrifugation in the blood storage bag 105.

In addition, the blood sampling bag 103, the blood storage bags 105 and 106, and the medical-solution-filled bag 107 each have the discharge ports 108 on its top side. In addition, the blood sampling bag 103, the blood storage bags 105 and 106, and the medical-solution-filled bag 107 each have attached thereto a label 109 to indicate the blood component to be placed therein. In addition, the tubes 101b to 101f are each provided with a non-illustrated channel sealing member, as necessary. A channel sealing member is a member that is installed in such a manner to block (seal) the channel of the tube and opens the channel upon breakage. Further, the blood sampling bag 103 is usually filled with an anticoagulant. As the anticoagulant, ACD solution, CPD solution, and the like are usable.

Incidentally, as the blood storage bag 105, a container made of the resin composition described above is used. As the blood sampling bag 103, the blood storage bag 106, and the medical-solution-filled bag 107, known containers are used. For example, a container made of polyvinyl chloride or the like and having a volume of about 100 to 600 mL is used. In addition, also as the blood sampling needle 102, a known metal needle is used, and also as the tubes 101a to 101f and the branch pipe 104, known resin tubes made of polyvinyl chloride or the like are used. Further, also as the blood processing filter 110, a known blood processing filter is used. Further, in the blood bag system 100, as long as the blood storage bag 105 to contain a red blood cell concentrate is made of the resin composition described above, the arrangements of the bags 103, 105, 106, and 107 and the blood processing filter 110 are not limited to the arrangements shown in Fig. 1, and other arrangements are also possible.

Examples

Per 100 parts by mass of polyvinyl chloride, a composite plasticizer composed of DiNCH and BTHC was incorporated in the amounts shown in Table 1, and also 8 parts by mass of epoxidized soybean oil, 0.048 parts by mass of zinc soap, and 0.064 parts by mass of calcium soap were incorporated, thereby producing each resin composition. Incidentally, the blending ratio between DiNCH and BTHC is as shown in Table 1.

[Table 1]

| | No. | Resin composition | | | Red blood cell storage stability | Pressure resistance |
|---|---|---|---|---|---|---|
| | | Composite plasticizer amount (part by mass) | DiNCH proportion (mass %) | BTHC proportion (mass %) | Hemolysis rate (%) | |
| Comparative Example | 1 | 66 | 0 | 100 | 1.30 | ○ |
| | 2 | 68 | 7 | 93 | 1.41 | ○ |
| Example | 3 | 33 | 15 | 85 | 1.20 | ○ |
| | 4 | 49 | 29 | 71 | 1.24 | ○ |
| | 5 | 40 | 50 | 50 | 1.19 | ○ |
| | 6 | 49 | 71 | 29 | 1.12 | ○ |
| Comparative Example | 7 | 91 | 77 | 23 | 1.23 | × |
| Example | 8 | 33 | 85 | 15 | 1.22 | ○ |
| | 9 | 68 | 93 | 7 | 1.27 | ○ |
| Comparative Example | 10 | 55 | 100 | 0 | 1.35 | ○ |

The resin composition was extruded through a T-die to produce a 0.4-mm-thick resin sheet. The produced two resin sheets were fused together. In this manner, bags for evaluation having a volume of 80 mL and a volume of 450 mL, respectively, were produced. Using the bags for evaluation produced, the red blood cell storage stability and the pressure resistance were evaluated according to the following procedure. Incidentally, the red blood cell storage stability was evaluated based on the hemolysis rate. The results are shown in Table 1.

(Red Blood Cell Storage Stability)

15 mL of a red blood cell storage solution (SAGM solution) and 60 mL of a red blood cell concentrate were mixed to give a red blood cell preparation. This red blood cell preparation was placed inside the bag for evaluation having a volume of 80 mL, and stored for six weeks at a temperature maintained at 4°C.
A specimen was collected from the red blood cell preparation after the completion of storage, and the hemoglobin concentration in the collected specimen and the hematocrit were measured with an automatic blood cell analyzer (XE-5000, manufactured by sysmex). In addition, using the red blood cell preparation after the completion of storage, the free hemoglobin concentration in the bloodplasma was calculated by the following LCV method.

(LCV Method)

(1) 20 mg of Leuco Crystal Violet (manufactured by SIGMA-ALDRICH) is dissolved in 20 mL of acetic acid, and then 75 mL of acetone and 25 mL of RO water are added, followed by stirring and mixing; this mixture is used as a coloring reagent.
(2) 30 mL of RO water is added to 1 mL of 30 mass% oxygenated water and mixed; this mixture is used as a chromogenic substrate solution.
(3) The red blood cell preparation after the completion of storage is centrifuged, and the supernatant is collected and used as a measurement sample for the free hemoglobin content in the bloodplasma. Inaddition, a hemoglobin standard solution (HEMOCON-N, manufactured by Alfresa Pharma) is diluted with RO water, whereby a hemoglobin standard solution for the preparation of a calibration curve (calibration curve sample) having a concentration of 2 to 300 mg/dL is also prepared at the same time.
(4) 6 mL of the coloring reagent, the measurement sample, and 25 μL of the calibration curve sample are added to a test tube and thoroughly stirred.
(5) Further, 1 mL of the chromogenic substrate solution is added and stirred, followed by incubation for 20 minutes in a hydraulic thermostat at 37°C.

(6) After the completion of incubation, the absorbance of 590 nm was measured using a spectrophotometer (V-650, manufactured by Jasco), and, at the same time, the free hemoglobin concentration in the blood plasma was calculated from the measured value of the calibration curve sample.

Using the hemoglobin concentration in the specimen, hematocrit, and free hemoglobin concentration in the bloodplasma measured and calculated above, the hemolysis rate (%) was calculated by the following equation (1). Incidentally, with respect to the calculated hemolysis rate (%), it can be said that the smaller the value is, the more the destruction of red cells is suppressed, indicating excellent storage stability for red blood cells.

[Mathematical Formula 1]

$$\text{Hemolysis rate (\%)} = \frac{\text{free hemoglobin concentration in the blood plasma (mg/dL)} \times (1 - \text{hematocrit}/100)}{\text{hemoglobin concentration in the specimen (g/dL)} \times 1000} \times 100 \ \ldots(1)$$

(Pressure Resistance)

Using the produced bag for evaluation having a volume of 450 mL, the following leakage experiments (1) to (3) in accordance with ISO 3826 were performed.

(1) 450 mL of water is injected into the bag for evaluation and centrifuged at 37°C for 10 minutes at 5000 G, and it is checked whether there is no leakage. Subsequently, at 23±5°C, the bag for evaluation is pressed between two plates until the internal pressure of the bag becomes 50 kPa higher than the atmospheric pressure. The bag is kept in that state for 10 minutes, and it is checked whether there is no leakage.
(2) 450 mL of water is injected into the bag for evaluation and centrifuged at 4°C for 10 minutes at 5000 G, and it is checked whether there is no leakage. Subsequently, at 23±5°C, the bag for evaluation is pressed between two plates until the internal pressure of the bag becomes 50 kPa higher than the atmospheric pressure. The bag is kept in that state for 10 minutes, and it is checked whether there is no leakage.
(3) 225 mL of water is injected into the bag for evaluation, slowly frozen, preserved at -80°C for 24 hours, and further immersed in water at 37±2°C for 60 minutes to return to room temperature. It is checked whether there is no leakage at this time.

When there was no leakage in all the (1) to (3), the pressure resistance was evaluated as excellent (○), while when there was leakage in any of the (1) to (3), the pressure resistance was evaluated as poor (×).
As shown in Table 1, in the examples that satisfy the requirements of the present invention (Nos. 3 to 6, 8, and 9), the red blood cell storage stability and pressure resistance were excellent as compared with the comparative examples that do not satisfy the requirements of the present invention (Nos. 1, 2, 7, and 10).
In Comparative Example (No. 1), because a plasticizer composed only of BTHC was used, the red blood cell storage stability was poor. In Comparative Example (No. 2), because the blending ratio between DiNCH and BTHC in the composite plasticizer does not satisfy the range specified in the present invention, the red blood cell storage stability was poor. In Comparative Example (No. 7), because the amount of composite plasticizer incorporated is more than the upper limit, the pressure resistance was poor. In Comparative Example (No. 10), because a plasticizer composed only of DiNCH was used, the red blood cell storage stability was poor.

Reference Signs List

100: Blood bag system
103: Blood sampling bag
105: Blood storage bag (blood storage container)
105A: Container main body
106: Blood storage bag
107: Medical-solution-filled bag
110: Blood processing filter

**Claims**

1.  A blood storage container comprising a container main body made of a resin composition incorporating, per 100 parts by mass of polyvinyl chloride, 30 to 70 parts by mass of a composite plasticizer combining a first plasticizer and a second plasticizer,
    the first plasticizer being
    di-isononyl-cyclohexane-1,2-dicarboxylate,
    the second plasticizer being a citrate,
    the blending ratio between the first plasticizer and the second plasticizer in the composite plasticizer being 15/85 to 93/7.

2.  The blood storage container according to claim 1, wherein the citrate is butyryl trihexyl citrate.

## INTERNATIONAL SEARCH REPORT

International application No.

PCT/JP2015/054445

### A. CLASSIFICATION OF SUBJECT MATTER
*A61J1/10*(2006.01)i

According to International Patent Classification (IPC) or to both national classification and IPC

### B. FIELDS SEARCHED

Minimum documentation searched (classification system followed by classification symbols)
A61J1/10

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | "DEHP Daitai Kasozai o Riyo shita Shinki Ketsueki Bag no Kaihatsu", Japanese Society for Biomaterials Symposium 2012 Yokoshu, 26 November 2012 (26.11.2012), page 362 | 1–2 |
| A | JP 6-506385 A (Baxter International Inc.), 21 July 1994 (21.07.1994), claim 20 & WO 1993/014810 A2 | 1–2 |
| P,X<br>A | JP 2014-223182 A (Kawasumi Laboratories, Inc.), 04 December 2014 (04.12.2014), claims 1 to 7 (Family: none) | 1<br>2 |

☐ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 19 May 2015 (19.05.15) | 26 May 2015 (26.05.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | |
| | Telephone No. |

Form PCT/ISA/210 (second sheet) (July 2009)

**EP 3 124 006 A1**

REFERENCES CITED IN THE DESCRIPTION

**Patent documents cited in the description**

- JP 61051044 A